# EUROPEAN PATENT APPLICATION

(11) **EP 1 582 588 A1**
(43) Date of publication of application: **05.10.2005**
(21) Application number: 04700504.6
(22) Date of filing: 07.01.2004
(51) Int. Cl.: C12N 15/09

(54) **PROCESS FOR PRODUCING siRNA**

(30) Priority: 08.01.2003 JP 2003002124
(71) Applicant: Suzuki, Tsutomu, Chiba 277-0882 (JP)
(72) Inventor: Suzuki, Tsutomu, Chiba 277-0882 (JP)
(74) Representative: Sternagel, Fleischer, Godemeyer & Partner
(86) International application number: PCT/JP2004/000046
(87) International publication number: WO 2004/063372

(57) **Abstract**

It is an object of the present invention to develop an inexpensive and simple method for transcription and synthesis of siRNA. The present invention provides an oligonucleotide, which at least comprises, in a direction from the 5'-terminus to the 3'-terminus:
(1) an antisense sequence of a target nucleic acid sequence;
(2) a trimming sequence which is cleaved with base-specific RNase;
(3) a sense sequence of a target nucleic acid sequence;
(4) an antisense sequence of a promoter sequence;
(5) a sequence that forms a loop; and
(6) a sense sequence of a promoter sequence,
wherein the above-described antisense sequence and sense sequence of a promoter sequence form a double strand in a molecule via a hairpin structure, and when DNA is transcribed, a transcriptional product from the above-described antisense sequence and sense sequence of a target nucleic acid sequence forms a double strand in a molecule via the trimming sequence.

## Description

### TECHNICAL FIELD

The present invention relates to a method for producing siRNA and an oligonucleotide used for the above method.

### BACKGROUND ART

### (1) Functional analysis of gene based on reverse genetics

The genome project has rapidly developed and has almost accomplished complete determination of the genomic DNA sequences of all organisms, including humans. According to a general outline of the sequence of the human genome published in the spring of 2001, the total number of human genes is estimated to be approximately 30,000 to 40,000. In the studies that have been conducted to date, some kind of functional analysis has been conducted on less than 10,000 types of genes, but the functions of the remaining 20,000 to 30,000 genes have been still unknown. In order to understand humans, it is necessary to clarify the functions of all human genes. In order to clarify the functions of a gene, it is important not only to identify a gene product encoded by the gene, that is, a protein or RNA, but also to clarify the expression control mechanism or the network with other genes. Molecular genetics is an effective means therefor. Examples of organisms for which molecular genetic means have been most developed include model organisms such as *Escherichia coli, Bacillus subtilis,* yeast, or nematodes. Molecular genetics have vigorously been applied for a long period of time to *Escherichia coli,* which is a representative example of the prokaryotes, and yeast, which is a representative example of the lower eukaryotes. In 1997, the entire genome nucleotide sequence of *Escherichia coli* and that of yeast were determined. The total number of genes of *Escherichia coli* and that of yeast are approximately 4,300 types and approximately 6,100 types, respectively. Of these, approximately 2,000 genes have been reported as genes with unknown functions in both cases of *Escherichia coli* and yeast. As a means for searching for these gene functions, a reverse genetic approach (knockout method) involving gene disruption is effective, when the target is a nonessential gene. In the case of yeast, a gene of interest can relatively easily be disrupted by homologous recombination using monoploid cells. With regard to approximately 5,000 types of nonessential genes of yeast, disrupted strains have been constructed since the early stage. Researchers over the world have used such disrupted strains as their study targets. A Japanese study team has begun a project concerning *Escherichia coli* as well, and a library of all gene disrupted strains will have been constructed in the near future.

### (2) Development of RNAi

The nematode is a model for multicellular organisms, the cell lineages of all of its less than 1,000 cells have been clarified, and the entire genome sequence thereof was determined in 1998. The presence of all 19,000 genes thereof has been clarified. Since large quantities of human genes are homologous to nematode genes, determination of the roles of these genes results in analysis of human genes.

In order to produce a gene deletion mutant nematode, a general gene disruption method that is commonly applied to *Escherichia coli* or yeast is not used. Rather, a gene expression suppression method (knockdown method) involving RNA interference (or RNAi) is used. RNAi is a phenomenon whereby suppression of gene expression takes place specifically to a target gene when cells are transfected with antisense RNA to a specific gene. In 1998, it was reported that the effect of suppressing gene expression is significantly improved by introduction of double-stranded RNA (dsRNA) (Fire, A., Xu, S., Montgomery, M. K., Kostas, S. A., Driver, S. E. and Mello, C. C. (1998) Potent and specific genetic interference by double-stranded RNA in Caenorhabditis elegans. *Nature,* 391, 806-11). Recently, a method for suppressing gene expression, not using antisense RNA, but using dsRNA, is called RNAi. Several methods have been developed. Among them, a method involving microinjection of dsRNA into a nematode egg is particularly frequently applied. Experiments regarding suppression of gene expression, in which all the genes have been targeted, have been conducted on a massive scale (Fraser, A.G., Kamath, R.S., Zipperlen, P., Martinez-Campos, M., Sohrmann, M. and Ahringer, J. (2000) Functional genomic analysis of C. elegans chromosome I by systematic RNA interference. *Nature,* 408, 325-30; and Gonczy, P., Echeverri, C., Oegema, K., Coulson, A., Jones, S. J., Copley, R. R., Duperon, J., Oegema, J., Brehm, M., Cassin, E., Hannak, E., Kirkham, M., Pichler, S., Flohrs, K., Goessen, A., Leidel, S., Alleaume, A. M., Martin, C., Ozlu, N., Bork, P. and Hyman, A. A. (2000) Functional genomic analysis of cell division in C. elegans using RNAi of genes on chromosome III. *Nature,* 408, 331-6). Thus, RNAi has entered the limelight as a reverse genetic tool for analyzing genes.

### (3) Action mechanism of RNAi

Analysis of a knockout mouse obtained by disrupting the homologous mouse genes is the most effective means for analyzing human genes in a reverse genetic manner. However, since a mammalian somatic cell genome is a diploid, a process of producing a homologous knockout mouse by mating chimeric mice having one chromosomal complement disrupted is required. Thus, since large degrees of manpower, cost, and time are required for disruption of a gene, such means does not satisfy the requirements for a comprehensive and rapid approach in the post-genome era. Since RNAi suppresses the expression of a gene at the transcription level, many researchers have studied the application of RNAi to mammalian cells since the early stage. However, application of RNAi to mammalian cells has been fundamentally problematic for the following reasons. That is, when long dsRNA is introduced into mammalian cells, as in the case of nematodes or flies, protein kinase (PKR) and 2',5'-oligoadenylate synthetase (2',5'-AS) are activated, and decomposition of mRNA that is non-specific to a nucleotide sequence and shut-down of protein synthesis thereby take place (Manche, L., Green, S. R., Schmedt, C. and Mathews, M.B. (1992) Interactions between double-stranded RNA regulators and the protein kinase DAI. *Mol Cell Biol,* 12, 5238-48; and Minks, M. A., West, D. K., Benvin, S. and Baglioni, C. (1979) Structural requirements of double-stranded RNA for the activation of 2',5'-oligo(A) polymerase and protein kinase of interferon-treated HeLa cells. *J Biol Chem,* 254, 10180-3). A key for solving this problem has been found in the analysis of the expression control mechanism of RNAi in nematodes. When dsRNA is introduced into cells, it is processed into short double-stranded RNA fragments with 21 to 23 mer by the action of an RNA cleavage enzyme specific to dsRNA, which is called Dicer, belonging to the RNase III family (Bernstein, E., Caudy, A. A., Hammond, S. M. and Hannon, G. J. (2001) Role for a bidentate ribonuclease in the initiation step of RNA interference. *Nature,* 409, 363-6; and Zamore, P. D., Tuschl, T., Sharp, P. A. and Bartel, D. P. (2000) RNAi: double-stranded RNA directs the ATP-dependent cleavage of mRNA at 21 to 23 nucleotide intervals. *Cell,* 101, 25-33). The antisense strand of each RNA fragment binds to the mRNA of a target, and a ribonuclease complex known as RISC (RNA-induced silencing complex) acts on the thus formed complex, so that the target is disrupted (Hammond, S. M., Bernstein, E., Beach, D. and Hannon, G. J. (2000) An RNA-directed nuclease mediates post-transcriptional gene silencing in Drosophila cells. *Nature,* 404, 293-6). Regarding nematodes, it has been known that once dsRNA is introduced into the egg thereof, duration of gene expression suppression is long, and that the effect of suppressing gene expression is maintained over generations. It has become clear that this is due to a mechanism whereby a larger amount of dsRNA is amplified by the action of RNA-dependent RNA replicase when an antisense strand binds to the mRNA of a target (degradative PCR) (Lipardi, C., Wei, Q. and Paterson, B. M. (2001) RNAi as random degradative PCR: siRNA primers convert mRNA into dsRNAs that are degraded to generate new siRNAs. *Cell,* 107, 297-307).

### (4) Application of siRNA to mammalian cells

Taking into consideration the aforementioned study results, the group of Tuschl has conceived of the introduction of short dsRNA from the initial stage to prevent the protective mechanism of mammalian cells against long dsRNA. Cultured human cells were transfected with dsRNA consisting of 21 bases having a nucleotide sequence complementary to a target gene at a low concentration such as several tens of nM. As a result, suppression of gene expression that is specific to the target gene was observed (Elbashir, S. M., Harborth, J., Lendeckel, W., Yalcin, A., Weber, K. and Tuschl, T. (2001a) Duplexes of 21-nucleotide RNAs mediate RNA interference in cultured mammalian cells. *Nature,* 411, 494-8). It has been reported that the length of RNA that is most effective for exhibiting such an expression-suppressing effect is 21 mer, and that dsRNA comprising 2 bases overhung at the 3'-terminus is preferable (Elbashir, S. M., Harborth, J., Lendeckel, W., Yalcin, A., Weber, K. and Tuschl, T. (2001 a) Duplexes of 21-nucleotide RNAs mediate RNA interference in cultured mammalian cells. *Nature,* 411, 494-8; and Elbashir, S. M., Lendeckel, W. and Tuschl, T. (2001b) RNA interference is mediated by 21- and 22-nucleotide RNAs. *Genes Dev,* 15, 188-200). Such short double-stranded RNA with a length of approximately 20 mer is generally called small interfering RNA (siRNA). To date, the knockout technique has been used for the functional analysis of genes. If a method using siRNA were established, significant reduction in the time necessary for experiments and in cost would be realized.

### (5) Organic synthetic method

In general, for production of short RNA consisting of 21 bases, RNA that is synthesized according to the phosphoamidite method based on organic chemistry has widely been used. Organic synthesis is advantageous in that it does not require selection of sequences (that is, synthesis can be carried out for RNA having any type of sequence). In addition, it has been known that when 2 bases overhung at the 3'-terminus are TT of DNA, the activity of suppressing gene expression is somewhat increased (Elbashir, S. M., Harborth, J., Lendeckel, W., Yalcin, A., Weber, K. and Tuschl, T. (2001a) Duplexes of 21-nucleotide RNAs mediate RNA interference in cultured mammalian cells. *Nature,* 411, 494-8). Thus, organic synthesis is advantageous also in that chimeric nucleic acid of DNA-RNA can be produced based on organic synthesis. At present, production on assignment of synthetic RNA is carried out, and organic synthesis of siRNA according to the phosphoamidite method is the most common production method at the present moment. However, such organic synthesis of RNA according to the phosphoamidite method is disadvantageous: in that synthesis of RNA by this method requires a longer reaction time than the case of synthesis of DNA, thus necessitating a long time for synthesis; in that deprotection requires a long time due to the presence of 2'-hydroxyl group-protecting groups; and in that this method requires considerable costs of production and quality control. Moreover, recent studies have discovered that the position of a complementary sequence consisting of 19 bases in a target gene that is set when siRNA is designed makes difference in the effect of suppressing gene expression. Thus, it is generally necessary to design siRNA at multiple sites, when a single gene is knocked down, and the supply of siRNA from synthetic RNA has a certain limit. Accordingly, it is difficult to say that the organic synthetic method is a general-purpose technique.

### (6) In vitro transcription and synthesis method

Hence, a method for producing siRNA by *in vitro* transcription reactions has recently become a focus of attention. Since RNA is enzymatically transcribed and synthesized using synthetic DNA as a template in such an *in vitro* transcription reaction, siRNA can be synthesized relatively inexpensively. In addition, it has been reported that the thus synthesized siRNA has a higher effect of suppressing gene expression than that of organic synthetic siRNA with the same design. Picard et al. have used T7 RNA polymerase to produce two RNA chains, a sense strand and an antisense strand, from two sets of DNA templates. They have converted the two RNA chains into double-stranded RNA, and have used it (Donze, O. and Picard, D. (2002) RNA interference in mammalian cells using siRNAs synthesized with T7 RNA polymerase. *Nucleic Acids Res,* 30, e46). When the initial base is G, T7 RNA polymerase can achieve efficient transcription. Although there is a certain restriction that the 5'-terminus of siRNA is inevitably G, the use of T7 RNA polymerase is advantageous in that it is much more inexpensive and exhibits higher activity than organic synthetic siRNA. Moreover, an siRNA production kit (Silencer siRNA construction kit) utilizing a transcription method has recently been released from Ambion. This is a method, which comprises: first producing two sets of template DNA used for a sense strand and an antisense strand; transcribing them to synthesize sense-strand RNA and antisense-strand RNA, using T7 RNA polymerase; converting them into a double-strand; and finally removing a single-stranded region by treating the RNA with RNase. The kit comprises synthetic DNA containing a T7 promoter used for production of templates, an enzyme and a reagent used for production of templates, T7 RNA polymerase and a reagent used for transcription, DNase and RNase used for digestion of templates and removal of a single-stranded region from RNA, a cartridge used for purification of siRNA, and the like. In addition to these items, two synthetic DNAs consisting of 29 bases used as templates should be prepared for a single design. When this kit is used, a total of three synthetic DNAs used for production of templates are required, and further this kit is problematic due to the presence of complicated reaction steps.

### DISCLOSURE OF THE INVENTION

It is an object of the present invention to solve the aforementioned problems of the prior art techniques. In other words, it is an object of the present invention to develop an inexpensive and simple method for transcription and synthesis of siRNA.

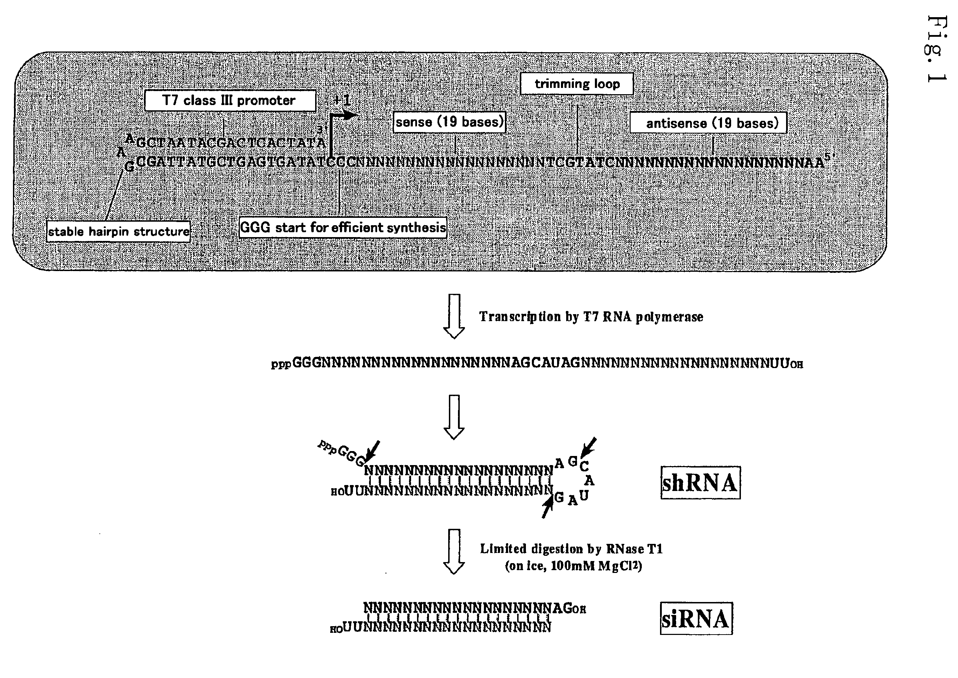

As a result of intensive studies directed towards achieving the aforementioned object, the present inventors have found that siRNA can be transcribed and synthesized in a simple and inexpensive manner by a method for transcription and synthesis of siRNA, the summary of which is shown in Figure 1 of the present specification, thereby completing the present invention.

That is to say, the present invention provides an oligonucleotide, which at least comprises, in a direction from the 5'-terminus to the 3'-terminus:
(1) an antisense sequence of a target nucleic acid sequence;
(2) a trimming sequence which is cleaved with base-specific RNase;
(3) a sense sequence of a target nucleic acid sequence;
(4) an antisense sequence of a promoter sequence;
(5) a sequence that forms a loop; and
(6) a sense sequence of a promoter sequence,
wherein the above-described antisense sequence and sense sequence of a promoter sequence form a double strand in a molecule via a hairpin structure, and when DNA is transcribed, a transcriptional product from the above-described antisense sequence and sense sequence of a target nucleic acid sequence forms a double strand in a molecule via the trimming sequence.

In another aspect, the present invention provides an oligonucleotide, which at least comprises, in a direction from the 5'-terminus to the 3'-terminus:
(1) an antisense sequence of a target nucleic acid sequence;
(2) a trimming sequence which is cleaved with base-specific RNase;
(3) a sense sequence of a target nucleic acid sequence; and
(4) an antisense sequence of a promoter sequence,
wherein, when DNA is transcribed, a transcriptional product from the above-described antisense sequence and sense sequence of a target nucleic acid sequence forms a double strand in a molecule via the trimming sequence.

In the above-described oligonucleotide, at least a promoter sequence region may be double-stranded.

In another aspect, the present invention provides double-stranded DNA, which consists of the above-described oligonucleotide and an oligonucleotide having a sequence complementary to the above-described oligonucleotide.

The oligonucleotide of the present invention preferably has two bases AA at the 5'-terminus located upstream of the antisense sequence of a target nucleic acid sequence.

In the oligonucleotide of the present invention, the trimming sequence which is cleaved with RNase is preferably represented by 5'-C(D)ₖCD-3' (wherein D represents A, T, or G, and k represents an integer between 0 and 100, wherein (k + 1) number of D bases may be identical to or different from one another).

In the oligonucleotide of the present invention, the trimming sequence which is cleaved with RNase is preferably represented by 5'-CTATGCT 3'.

In the oligonucleotide of the present invention, -CCC- preferably exists between the sense sequence of a target nucleic acid sequence described in (3) above and the antisense sequence of a promoter sequence described in (4) above.

The promoter sequence is preferably a T7 class III promoter sequence.

The sequence that forms a loop described in (5) above is preferably a sequence comprising -GNA- (wherein N represents A, T, C, or G).

The oligonucleotide of the present invention is preferably an oligonucleotide represented by 5'-AA-(the antisense sequence of a target nucleic acid sequence)-CTATGCT-(the sense sequence of a target nucleic acid sequence)-CCC-TATAGTGAGTCGTATTA-GCGAAGC-TAATACGACTCACTATA-3' .

In another aspect, the present invention provides a method for producing shRNA, which comprises transcribing DNA, using the above-described oligonucleotide of the present invention as a template and using RNA polymerase.

Such transcription is preferably carried out *in vitro.*

T7 RNA polymerase is preferably used as RNA polymerase.

In another aspect, the present invention provides shRNA produced by the above-described method.

In another aspect, the present invention provides a method for producing siRNA, which comprises treating the shRNA produced by the above-described method with base-specific RNase.

In another aspect, the present invention provides a method for producing siRNA, which comprises transcribing DNA using the oligonucleotide of the present invention as a template and using RNA polymerase, so as to produce shRNA, and then treating the shRNA with base-specific RNase.

In another aspect, the present invention provides a method for suppressing the expression of a gene containing a target nucleic acid sequence by RNAi, using the shRNA or siRNA produced by the method of the present invention.

In another aspect, the present invention provides a reagent kit for carrying out the method of the present invention, which comprises RNA polymerase and base-specific RNase.

### BRIEF DESCRIPTION OF THE DRAWINGS

Figure 1 shows the summary of an example of the method of transcription and synthesis of siRNA of the present invention.
Figure 2 shows a comparison between the activities of siRNA and shRNA targeting a lamin A/C protein. HeLa cells were used. The HeLa cells were knocked down with siRNA or shRNA (each 50 nM) acting on lamin A/C. Thereafter, Western blotting analysis was carried out using an anti-lamin A/C antibody.
Figure 3 shows a comparison between the activities of siRNA and shRNA targeting a lamin A/C protein. Both SiRNA and shRNA synthesized by transcription exhibit their effects at lower concentrations than that of organic synthetic siRNA.

### BEST MODE FOR CARRYING OUT THE INVENTION

### (1) Oligonucleotide

The oligonucleotide of the present invention at least comprises, in a direction from the 5'-terminus to the 3'-terminus:
(1) an antisense sequence of a target nucleic acid sequence;
(2) a trimming sequence which is cleaved with base-specific RNase;
(3) a sense sequence of a target nucleic acid sequence;
(4) an antisense sequence of a promoter sequence;
(5) a sequence that forms a loop; and
(6) a sense sequence of a promoter sequence.

The term "target nucleic acid sequence" is used to mean a nucleic acid sequence existing in a gene whose expression is intended to be suppressed. Any given gene can be used as a gene whose expression is intended to be suppressed. A gene that has been cloned, but that has functions that remain unknown, is also included in such a target gene. Otherwise, a target gene may be a gene of a foreign reporter protein or a gene of a mutant protein thereof. When the nucleic acid sequence of such a foreign reporter protein gene or a mutant protein gene thereof is used, RNAi effects can easily be detected and evaluated by the technique of the present invention. The length of a target nucleic acid sequence is not particularly limited, as long as a transcriptional product exhibits desired RNAi effects. It is generally between approximately 10 and 50 bases, preferably between approximately 10 and 30 bases, and more preferably between approximately 15 and 25 bases. The length of the sense sequence of a target nucleic acid sequence is preferably identical to the length of the antisense sequence thereof. However, their lengths may be somewhat different, as long as a transcriptional product exhibits desired RNAi effects.

The term "trimming sequence which is cleaved with RNase" is used to mean a nucleotide sequence that can be cleaved with base-specific RNase.

The type of base-specific RNase is not particularly limited. For example, RNase T1 is an example of G-specific RNase. RNase U2 is an example of A- and G-specific RNase. RNase CL3 is an example of C-specific RNase. RNase A and RNase I are examples of C- and U-specific RNase. RNase PhyM is an example of A- and U-specific RNase.

When G-specific RNase such as RNase T1 is used, for example, a trimming sequence can be designed to contain G. Specifically, a trimming sequence represented by 5'-C(D)ₖCD-3' (wherein D represents A, T, or G, and k represents an integer between 0 and 100, wherein (k + 1) number of D bases may be identical to or different from one another) can be used. An example of such a trimming sequence is 5'-CTATGCT-3'.

Likewise, in the case of A- and G-specific RNase, a trimming sequence represented by 5'-Y(R)ₖYR-3' can be used, and in the case of C-specific RNase, a trimming sequence represented by 5'-G(H)ₖGH-3' can be used. In the case of C- and U-specific RNase, a trimming sequence represented by 5'-R(Y)ₖRY-3' can be used, and in the case of A- and U-specific RNase, a trimming sequence represented by 5'-S(W)ₖSW-3' can be used. Herein, Y represents C or T; R represents A or G; H represents A, C, or T; S represents C or G; and W represents A or T.

A promoter sequence used in the present invention is not particularly limited, as long as it is a promoter of RNA polymerase. Examples of a promoter sequence used herein may include a T7 class III promoter sequence, an SP6 promoter sequence, and a T3 promoter sequence.

A sequence that forms a loop in the present invention preferably contains -GNA-(wherein N represents A, T, C, or G). Using such a sequence, the antisense sequence of a promoter sequence and the sense sequence thereof form a double strand in a molecule via a hairpin structure in the oligonucleotide of the present invention (Yoshizawa S et al., GNA trinucleotide loop sequences producing extraordinarily stable DNA minihairpins. Biochemistry. 1997 Apr 22; 36(16): 4761-7). Because of this structure, transcription can be carried out using the oligonucleotide of the present invention as a template and also using RNA polymerase, so as to synthesize RNA having an inverted repeat sequence of a target nucleic acid sequence. Moreover, when transcription is carried out using RNA polymerase, a transcriptional product obtained from the antisense sequence of a target nucleic acid sequence and the sense sequence thereof forms a double strand in a molecule via a trimming sequence.

In another aspect, the present invention provides an oligonucleotide, which at least comprises, in a direction from the 5'-terminus to the 3'-terminus:
(1) an antisense sequence of a target nucleic acid sequence;
(2) a trimming sequence which is cleaved with base-specific RNase;
(3) a sense sequence of a target nucleic acid sequence; and
(4) an antisense sequence of a promoter sequence,
wherein, when DNA is transcribed, the above-described antisense sequence and sense sequence of a target nucleic acid sequence form a double strand in a molecule via the trimming sequence. In this case, since a promoter sequence is only a single strand (antisense strand), an oligonucleotide having the sense sequence of the promoter sequence may be added thereto separately, so as to form a double strand, and thereafter, transcription may be carried out using RNA polymerase.

In another aspect of the present invention, a nucleotide sequence may be present on the 3'-terminal side after the sense sequence of a promoter sequence described in (6) above. For example, a nucleotide sequence complementary to each of the sense sequence of a target nucleic acid sequence and the antisense sequence thereof, may exist.

In addition, in a preferred aspect of the present invention, two bases AA can be added to the 5'-temrinus of the oligonucleotide (that is, upstream of the antisense sequence of a target nucleic acid sequence).

Moreover, in a preferred aspect of the present invention, a nucleotide sequence -CCC- can be inserted between the sense sequence of a target nucleic acid sequence and the antisense sequence of a promoter sequence. RNA synthesis efficiency with RNA polymerase can be increased by insertion of such a sequence.

An oligonucleotide represented by 5'-AA-(the antisense sequence of a target nucleic acid sequence)-CTATGCT-(the sense sequence of a target nucleic acid sequence)-CCC-TATAGTGAGTCGTATTA-GCGAAGC-TAATACGACTCACTATA-3' is an example of the oligonucleotide of the present invention. Figure 1 shows a case where a target nucleic acid sequence consists of 19 bases.

Particularly preferred examples of the present invention will be further described below with reference to Figure 1. It has been known that in an *in vitro* transcription reaction using T7 RNA polymerase, a template DNA region encoding RNA to be synthesized may be a single strand, and that only a promoter portion should be double-stranded (Milligan, J. F. and Uhlenbeck, O. C. (1989) Synthesis of small RNAs using T7 RNA polymerase. *Methods Enzymol,* **180,** 51-62). Thus, single-stranded synthetic DNA consisting of 91 bases as shown in Figure 1 can be used as template DNA. RNA to be synthesized by transcription is shRNA (small hairpin RNA), which has a structure wherein a loop of 7 bases (trimming loop) exists between a sense strand-an antisense strand of 19 bases. Two bases UU are overhung at the 3'-terminus of the antisense strand. Since the class III promoter region of T7 RNA polymerase should be a double strand, a hairpin structure in which DNA is stable due to the GAA triloop loop was introduced (Hirao, I., Kawai, G., Yoshizawa, S., Nishimura, Y., Ishido, Y., Watanabe, K. and Miura, K. (1994) Most compact hairpin-turn structure exerted by a short DNA fragment, d(GCGAAGC) in solution: an extraordinarily stable structure resistant to nucleases and heat. *Nucleic Acids Res,* **22**, 576-82). Thus, it is predicted that the promoter region of this template DNA will certainly have a double strand structure in an aqueous solution, as shown in Figure 1. An efficient GGG sequence was designed as a transcription initiation site. With regard to this template consisting of 91 bases, only 19 bases that constitute a sense strand-an antisense strand may be arranged in any given sequence, and the remaining 53 bases are all determined. This template is characterized in that single-stranded synthetic DNA directly functions as a template of shRNA. Taking into consideration the method of XXX et al. and the fact that the transcription kit available from Ambion uses three DNA portions and requires a step of producing a template using DNA polymerase (Klenow enzyme), the present method is extremely simple and rational. The transcribed shRNA forms a double-stranded portion consisting of 19 bases from a sense strand-an antisense strand, and has a trimming loop site AGCAUAG (XGXXXXG). The shRNA has a single-stranded portion GGG at the 5'-terminus thereof and a single-stranded overhung portion UU at the 3'-terminus thereof. The trimming loop is characterized in that it is a loop consisting of approximately 4 to 10 bases. It is necessary that G be located at the 2^{nd} position from the 5'-terminal side as the entrance of the loop, and that another G be located at the 3'-terminus of the loop. Next, shRNA is converted into siRNA in one step by limited digestion with ribonuclease T1. After completion of the transcription reaction, magnesium ions (MgCl₂ etc.) are directly added to the reaction solution to a final concentration of 50 to 100 mM. Thereafter, the reaction solution is preincubated on ice (0°C) for 10 minutes. Subsequently, ribonuclease T1 (RNase T1) used for G-specific cleavage is added to the resultant, and the mixture is then reacted on ice (0°C) for 30 to 60 minutes. By this treatment, a GGG sequence at the 5'-terminus and 5 bases CAUAG in a trimming loop are specifically removed. Two bases are overhung at the 3'-terminus of both a sense strand and an antisense strand of the obtained siRNA. Thus, using the above designed template, siRNA having any given target sequence can simply and efficiently be synthesized.

### (2) Production of shRNA and siRNA, and RNAi using the same

As described in (1) above with reference to Figure 1, shRNA can be produced by transcription using the oligonucleotide of the present invention as a template and using RNA polymerase. The shRNA produced by this method is novel in that it comprises a trimming sequence that is cleaved with base-specific RNase in a hairpin portion thereof. Such shRNA itself is included in the scope of the present invention.

In the method of the present invention, transcription can be carried out *in vitro.* In addition, T7 RNA polymerase, SP6 RNA polymerase, or T3 RNA polymerase can be used as T7RNA polymerase. Of these, T7 RNA polymerase is preferably used.

A transcription reaction using RNA polymerase can be carried out by common methods that have already been known to persons skilled in the art. For example, such a reaction can be carried out by adding magnesium chloride, NTP, spermidine, and dithiothreitol to a solution containing an oligonucleotide as a template, and finally adding T7 RNA polymerase thereto to an appropriate concentration. In order to eliminate pyrophosphoric acid generated as a by-product from the reaction solution, thereby promoting the transcription reaction, pyrophosphatase is preferably added. The transcription reaction can be carried out by incubating the thus obtained reaction mixture at 37°C for 60 minutes.

Thereafter, the shRNA produced by the aforementioned method is treated with base-specific RNase, so that the trimming sequence can be cleaved and that siRNA can be produced. As such base-specific RNase, RNase T1 that cleaves the sequence specifically at G, RNase CL3 that cleaves the sequence specifically at C, or the like can be used. From the viewpoint of cleavage of only a single-stranded loop portion, the treatment with RNase is preferably carried out in the presence of a high concentration of salts, such as in the presence of 100 mM MgCl₂.

Purification of shRNA or siRNA can be carried out as follows. First, proteins are eliminated from a reaction product by a common method such as a treatment with phenol or a treatment with chloroform. Thereafter, the resultant is loaded on 15% polyacrylamide gel and electrophoresed, and only the band portion of shRNA or siRNA of interest is cut out. Subsequently, the obtained gel is ground down, and the resultant is then immersed in a suitable solution (for example, a solution containing 0.5 M sodium chloride, 0.1% SDS, and 1 mM EDTA). It is then stirred at 37°C for a certain period of time, so as to elute RNA from the gel. After the gel has been eliminated from the solution, RNA is precipitated by addition of ethanol. A pellet is dissolved in a small amount of pure water and recovered, so as to obtain purified DNA of interest.

Using the shRNA or siRNA produced by the aforementioned method of the present invention, the expression of a gene containing a target nucleic acid sequence can be suppressed by RNAi.

When cultured cells such as HeLa cells are used, for example, siRNA or shRNA is mixed with a suitable transfection reagent (for example, OLIGOFECTAMINE, etc.), and the mixture is then added to the cultured cells, so that the cultured cells can be transfected with the siRNA or shRNA. When the cultured cells are cultured under preferred conditions, the RNAi effects take place in the cells, and the expression of a gene containing a target nucleic acid sequence is suppressed. Suppression of the gene expression can be confirmed by RT-PCR, Northern blotting, Western blotting, or the like. In addition, when the functions of a gene whose expression is suppressed have been clarified, suppression of the gene expression can also be confirmed by observing cell phenotype.

### (3) Reagent kit

The present invention also provides a reagent kit containing RNA polymerase and base-specific RNase, which is used for carrying out the aforementioned method of the present invention. The reagent kit of the present invention may comprise other reagents and/or a buffer solution that are necessary for carrying out a reaction using the above enzyme.

For example, a reaction system for an enzyme reaction using RNA polymerase may comprise magnesium chloride, NTP, spermidine, dithiothreitol, and pyrophosphatase, in addition to RNA polymerase. Thus, all of these reagents or several thereof may be contained in the reagent kit of the present invention.

The present invention will be more specifically described in the following examples. However, these examples are not intended to limit the scope of the present invention.

### EXAMPLES

### [1] Purpose

The activities of siRNA and shRNA produced by the method of the present invention are confirmed by knockdown of a lamin A/C protein. Moreover, a comparison among the above activities and the activity of organically synthesized siRNA is also made.

### [2] Experimental method

### Design of template DNA

DNA used as a template in synthesis of shRNA by transcription was produced by organic synthesis according to the phosphoamidite method (Hokkaido System Science Co., Ltd.). The length of template DNA was 91 bases, and such template DNA was directly used in the form of a single strand. The sequence thereof is 5'-AAC TGG ACT TCC AGA AGA ACA CTA TGC TTG TTC TTC TGG AAG TCC AGC CCT ATA GTG AGT CGT ATT AGC GAA GCT AAT ACG ACT CAC TAT A-3' (SEQ ID NO: 1). The sequence of this template DNA was designed such that the T7 RNA polymerase promoter region (5'-TAA TAC GAC TCA CTA TA-3') (SEQ ID NO: 2) was complementarily located at two sites on the 3'-terminal side, and such that the promoter region became a double strand by folding the portion in a hairpin shape. With this structure, recognition with T7 RNA polymerase became possible without preparing two synthetic DNAs, thereby achieving cost reduction. Moreover, in order to more stably convert the T7 promoter region into a double strand, a GAA triloop having the effect of stabilizing a hairpin structure was introduced between the two T7 promoter sequences (sense and antisense). It is expected that the efficiency of a transcription reaction with T7 RNA polymerase will be improved by introduction of the GAA triloop.

On the other hand, a sequence portion consisting of 19 bases (5'-CTG GAC TTC CAG AAG AAC A-3') (SEQ ID NO: 3) from a DNA sequence encoding a human lamin A/C protein was disposed on the 5'-terminal side of the template DNA, in the order of sense and antisense from the 5'-terminus. Thereafter, a trimming loop (CTATGCT) that would be a target site of limited digestion with ribonuclease T1 was disposed between the sense strand and the antisense strand. By disposition of such a trimming loop, a transcriptional product obtained by transcription with T7 RNA polymerase became RNA (shRNA) having a hairpin structure containing a trimming loop (AGCAUAG).

### Synthesis of shRNA by in vitro transcription method

For synthesis of shRNA by transcription, magnesium chloride, NTP, spermidine, and dithiothreitol were added to the above-described template DNA with a concentration 50 nM at pH 7.8. Finally, T7 RNA polymerase was added thereto to a final concentration of 0.02 µg/ml, and the reaction was allowed to initiate. In order to eliminate pyrophophoric acid generated as a by-product from the reaction solution, thereby promoting a transcription reaction, yeast-derived pyrophosphatase (SIGMA) was added thereto to a final concentration of 0.47 µg/ml. The obtained mixture was incubated at 37°C for 60 minutes, so as to conduct a transcription reaction. The reaction mixture was subjected to 12% polyacrylamide gel electrophoresis, so as to confirm generation of shRNA of interest.

### Generation of siRNA by limited digestion of shRNA (trimming)

Magnesium chloride was added to the above transcription reaction solution to a final concentration of 100 mM, and the mixture was then preincubated on ice (0°C) for 10 minutes. Subsequently, ribonuclease T1 (SIGMA) used for G-specific cleavage was added thereto to a final concentration of 30 µg/ml. The mixture was then reacted on ice (0°C) for 30 minutes. By this treatment, portions adjacent to two G bases on the 3'-terminal side in the trimming loop were cleaved to a limited extent, so as to eliminate only the sequence portion CAUAG. Because of low temperature and high magnesium concentration, the portion containing a double strand was not cleaved, although it contained G in the sequence thereof. This reaction mixture was subjected to 15% polyacrylamide gel electrophoresis, so as to confirm generation of siRNA of interest.

### Purification

Phenol treatment was performed twice on the above reaction solution, and chloroform treatment was then performed once thereon, so as to eliminate proteins. Thereafter, the resultant solution was loaded on 15% polyacrylamide gel and electrophoresed, and only the band portion of siRNA of interest was cut out using a cutter. Subsequently, the obtained gel was ground down, and the resultant was then immersed in a solution containing 0.5 M sodium chloride, 0.1% SDS, and 1 mM EDTA. The obtained solution was then stirred at 37°C for 12 hours, so as to elute RNA from the gel. After the gel had been eliminated from the solution, ethanol was added thereto, so as to precipitate RNA. A pellet was dissolved in a small amount of pure water and recovered.

### Knockdown

HeLa cells (1 x 10⁴ cells) were inoculated on a 48-well dish, and they were then cultured in 0.2 ml of medium (D-MEM + 10% FBS) overnight (30% to 35% confluent). Thereafter, 25 µl of OPTI-MEM I (GIBCO) was added to 7.4 fmol to 7.4 pmol of siRNA and shRNA corresponding to lamin A/C. At the same time, 1 µl of OLIGOFECTAMINE™ (Invitrogen) was diluted with 5 µl of OPTI-MEM I, and the dilution was left at room temperature for 10 minutes. Thereafter, the two solutions were slowly mixed with each other, and the obtained mixture was further left at room temperature for 20 minutes. The cells, which had been cultured overnight, were washed with OPTI-MEM. Thereafter, 120 µl of OPTI-MEM was added to each well. 30 µl of a mixed solution of siRNA and Oligofectamine was added thereto, and the resultant was slowly mixed. The obtained mixture was incubated in a CO₂ incubator at 37°C for 4 hours. Thereafter, 225 µl of a medium (DMEM + 30% FBS) was added thereto, and the obtained mixture was further cultured for 40 hours. Thereafter, the medium was removed, and 50 µl of a sample buffer used for 2 x SDS-PAGE was added to the cells and dissolved therein. The resultant product was transferred to an Eppendorf tube. It was subjected to sonication for 10 seconds x 5 times on ice, and was then heated at 100°C for 1 minute, so as to prepare a sample used for SDS-PAGE.

### Western blotting

30 µl of the sample was subjected to 10% SDS polyacrylamide gel electrophoresis. A PVDF membrane (Immobilon™-P Transfer Membrane, 0.45 µm, MILLIPORE) with a size of 6 x 9 cm was immersed in 100% methanol for 2 minutes. Thereafter, the membrane was stirred in a transcription buffer solution (obtained by mixing 3.03 g of Tris, 14.4 g of Glycine, 0.1 g of SDS, and 100 ml of MeOH into MilliQ, to a total amount of 1 L) at room temperature for 30 minutes. The gel obtained after the electrophoresis was set to a semi-dry type blotter, and the membrane was placed thereon. A paper filter (3 MM, Whatman) wetted with a transcription buffer solution was then placed thereon. Transcription was carried out at room temperature at 400 mA for 90 minutes. After completion of the transcription, the membrane was immersed in 10% skimmed milk (TBS), and blocking was carried out at 37°C for 1 hour. Thereafter, the membrane was allowed to react with a 100-times-diluted anti-lamin A/C primary antibody (Lamin A/C (636): sc-7292, Santa Cruz Biotechnology) (room temperature, 1 hour). As a control, an anti-tubulin antibody (MONOCLONAL ANTI-β-TUBULIN: T5293, SIGMA) was used. After completion of the reaction, the membrane was washed with TBS 3 times, and it was then allowed to react with a 1,500-times-diluted secondary antibody (Peroxidase-Conjugated Rabbit Anti-Mouse Immunogloulins:P0161, DAKO) (room temperature, 1 hour). The resultant membrane was washed with TBS 3 times, and was then allowed to emit light with ECL. Lamin A/C was quantified by autoradiography.

### [3] Results and consideration

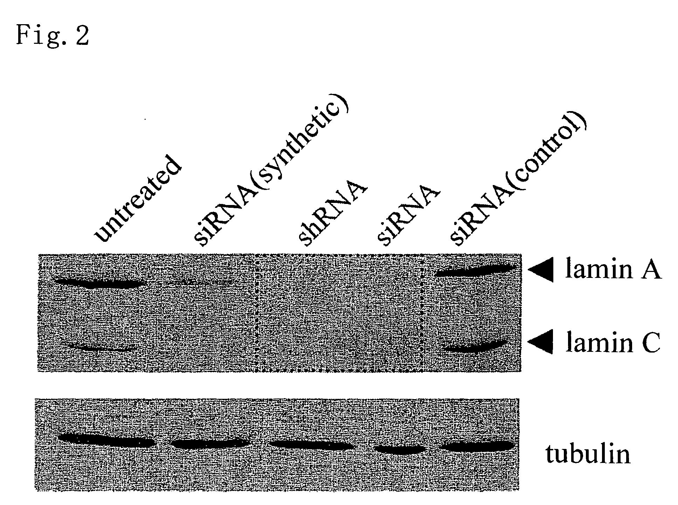

The results regarding 50 nM siRNA and 50 nM shRNA are shown in Figure 2. From the facts that tubulin emitted the same level of light in all cells and that knockdown of lamin A/C was not observed in siRNA to another target used as a control, the experiment is considered to have been successful. Both the siRNA and shRNA produced by transcription exhibited higher activity than that of the siRNA produced by organic synthesis.

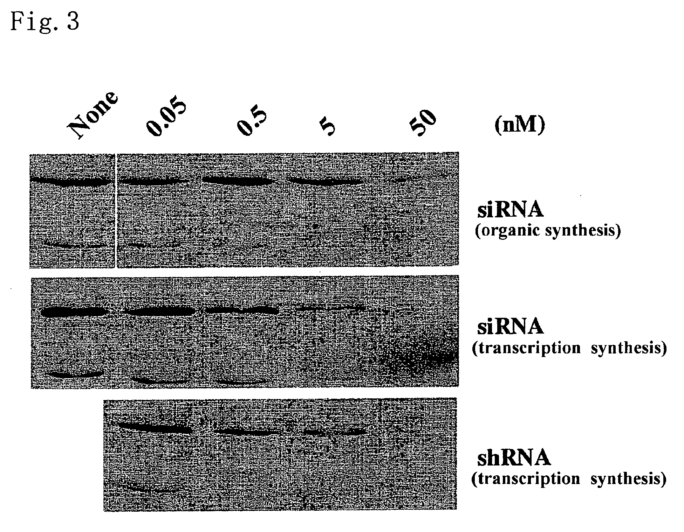

Moreover, Figure 3 shows data obtained by knockdown of lamin A/C while changing the concentration of siRNA. These results also show that the siRNA and shRNA produced by transcription exhibited higher activity than that of the siRNA produced by organic synthesis, and that the knockdown activity was observed from a concentration of approximately 5 nM.

### INDUSTRIAL APPLICABILITY

The use of the oligonucleotide of the present invention enables inexpensive and simple synthesis of siRNA by transcription. In particular, the present invention is advantageous in the following respects:
(1) production of only one template DNA (which consists of 91 bases in the present examples) is required;
(2) a determined sequence can be used, except for bases that constitute a target nucleic acid sequence;
(3) since previously synthesized bases can be used as initial bases (44 bases in the present examples), the time required for synthesis is reduced, and the cost of template DNA is significantly reduced;
(4) synthetic DNA can directly be used for transcription;
(5) transcription and synthesis can be carried out with only one tube (can be carried out on a 96-well plate);
(6) since the transcribed RNA is shRNA, it can directly be used;
(7) conversion of shRNA into siRNA can be carried out in one step;
(8) since sense and antisense are not transcribed separately, an annealing operation is unnecessary; and
(9) since no single-stranded RNA is generated, the yield of siRNA is high.

## Claims

1. An oligonucleotide, which at least comprises, in a direction from the 5'-terminus to the 3'-terminus:
(1) an antisense sequence of a target nucleic acid sequence;
(2) a trimming sequence which is cleaved with base-specific RNase;
(3) a sense sequence of a target nucleic acid sequence;
(4) an antisense sequence of a promoter sequence;
(5) a sequence that forms a loop; and
(6) a sense sequence of a promoter sequence,
wherein the above-described antisense sequence and sense sequence of a promoter sequence form a double strand in a molecule via a hairpin structure, and when DNA is transcribed, a transcriptional product from the above-described antisense sequence and sense sequence of a target nucleic acid sequence forms a double strand in a molecule via the trimming sequence.

2. An oligonucleotide, which at least comprises, in a direction from the 5'-terminus to the 3'-terminus:
(1) an antisense sequence of a target nucleic acid sequence;
(2) a trimming sequence which is cleaved with base-specific RNase;
(3) a sense sequence of a target nucleic acid sequence; and
(4) an antisense sequence of a promoter sequence,
wherein, when DNA is transcribed, a transcriptional product from the above-described antisense sequence and sense sequence of a target nucleic acid sequence forms a double strand in a molecule via the trimming sequence.

3. The oligonucleotide according to claim 2 wherein at least a promoter sequence region is double-stranded.

4. A double-stranded DNA, which consists of the oligonucleotide of claim 2 and an oligonucleotide having a sequence complementary to said oligonucleotide.

5. The oligonucleotide according to any of claims 1 to 4 which has two bases AA at the 5'-terminus located upstream of the antisense sequence of a target nucleic acid sequence.

6. The oligonucleotide according to any of claims 1 to 5 wherein the trimming sequence which is cleaved with RNase is represented by 5'-C(D)ₖCD-3' wherein D represents A, T, or G, and k represents an integer between 0 and 100, wherein (k + 1) number of D bases may be identical to or different from one another.

7. The oligonucleotide according to any of claims 1 to 6 wherein the trimming sequence which is cleaved with RNase is represented by 5'-CTATGCT-3'.

8. The oligonucleotide according to any of claims 1 to 7 wherein -CCC- exists between the sense sequence of a target nucleic acid sequence described in (3) and the antisense sequence of a promoter sequence described in (4).

9. The oligonucleotide according to any of claims 1 to 8 wherein the promoter sequence is a T7 class III promoter sequence.

10. The oligonucleotide according to any of claims 1 to 9 wherein the sequence that forms a loop described in (5) is a sequence comprising -GNA- wherein N represents A, T, C, or G..

11. An oligonucleotide represented by 5'-AA-(the antisense sequence of a target nucleic acid sequence)-CTATGCT-(the sense sequence of a target nucleic acid sequence)-CCC-TATAGTGAGTCGTATTA-GCGAAGC-TAATACGACTCACTATA-3'.

12. A method for producing shRNA, which comprises transcribing DNA, using the oligonucleotide or DNA of any of claims 1 to 11 as a template and using RNA polymerase.

13. The method for producing shRNA according to claim 12 wherein the transcription is carried out *in vitro.*

14. The method for producing shRNA according to claim 12 or 13 wherein T7 RNA polymerase is used as RNA polymerase.

15. shRNA produced by the method of any of claims 12 to 14.

16. A method for producing siRNA, which comprises treating the shRNA produced by the method of any of claims 12 to 14 with base-specific RNase.

17. A method for producing siRNA, which comprises transcribing DNA using the oligonucleotide of any of claims 1 to 11 as a template and using RNA polymerase, so as to produce shRNA, and then treating the shRNA with base-specific RNase.

18. A method for suppressing the expression of a gene containing a target nucleic acid sequence by RNAi, using the shRNA produced by the method of any of claims 12 to 14 or siRNA produced by the method of claim 16 or 17.

19. A reagent kit for carrying out the method of any of claims 12 to 14 and 16 to 18 which comprises RNA polymerase and base-specific RNase.


SEQUENCE LISTING
<110> iGENE
<120> A method for preparing siRNA
<130> A31662A
<160> 3
<210> 1
<211> 91
<212> DNA
<213> Artificial Sequence
<220>
<223> Description of Artificial Sequence: Synthetic DNA
<400> 1
aactggactt ccagaagaac actatgcttg ttcttctgga agtccagccc tatagtgagt 60
cgtattagcg aagctaatac gactcactat a 91
<210> 2
<211> 17
<212> DNA
<213> Artificial Sequence
<220>
<223> Description of Artificial Sequence: Synthetic DNA
<400> 2
taatacgact cactata 17
<210> 3
<211> 19
<212> DNA
<213> Artificial Sequence
<220>

<223> Description of Artificial Sequence: Synthetic DNA

<400> 3

ctggacttcc agaagaaca 19